# EUROPÄISCHE PATENTSCHRIFT

(11) **EP 2 493 383 B1**
(45) Veröffentlichungstag und Bekanntmachung des Hinweises auf die Patenterteilung: **16.12.2015**
(21) Anmeldenummer: 10782529.1
(22) Anmeldetag: 21.10.2010
(51) Int. Cl.: A61B 6/04, B29C 70/04

(54) **PATIENTENLIEGE UND VERFAHREN ZU DEREN HERSTELLUNG**
PATIENT TABLE AND METHOD FOR THE PRODUCTION THEREOF
TABLE MÉDICALE ET PROCÉDÉ POUR SA FABRICATION

(30) Priorität: 27.10.2009 DE 102009051048
(43) Veröffentlichungstag der Anmeldung: 05.09.2012
(73) Patentinhaber: Schmuhl Faserverbundtechnik GmbH & Co. KG, 07368 Liebschütz (DE)
(72) Erfinder: SCHMUHL, Peter, 07368 Liebschütz (DE); WEINHOLDT, Michael, 07751 Jena (DE); WEINZIERL, Uwe, 07743 Jena (DE); BACKER, J. A. D., NL-5491 St. Oedenrode (NL)
(74) Vertreter: Oehmke, Volker
(86) Internationale Anmeldenummer: PCT/DE2010/050079
(87) Internationale Veröffentlichungsnummer: WO 2011/050794

(56) Entgegenhaltungen:
- EP-A2- 0 433 272
- WO-A1-2011/001174
- WO-A2-2007/035920

## Beschreibung

### Technisches Gebiet

Die vorliegende Erfindung betrifft eine Patientenliege für diagnostische und medizinische Behandlungsgeräte.

### Stand der Technik

In der Medizintechnik werden Patientenliegen vielfältig im Zusammenhang mit diagnostischen Geräten oder Behandlungsgeräten eingesetzt. Insbesondere bei bildgebenden Untersuchungen, wie z. B. der Magnetresonanzspektroskopie (MR) oder der Computertomographie (CT) werden an die Patientenliegen hohe Anforderungen an die Festigkeit und Steifigkeit gestellt. Das ist notwendig, damit die Patienten während der Untersuchungszeit, die von mehreren Minuten bis zu einer Stunde dauern können, stabil in einer Position gelagert werden, um die Untersuchungsergebnisse nicht zu verfälschen oder gar unbrauchbar werden zu lassen. Ebenso müssen sie fest genug sein, damit sie bei Belastungen durch den Patienten nicht brechen. Die hohe Festigkeit und Steifigkeit müssen derartige Patientenliegen bereits bei geringen Wandstärken besitzen, weil die verfügbaren Räume zum Einbau der Patentenliegen in diagnostische Geräte häufig sehr klein sind und das Gewicht der Liege möglichst gering sein soll. Patientenliegen aus Thermoplasten, wie sie in der DE 44 16 202 A beschrieben sind, erfüllen die Forderungen nach einer hohen Steifigkeit aufgrund des geringen E-Moduls der Thermoplaste von maximal 4 GPa nicht.

Darüber hinaus sollen die Patientenliegen das medizinische Untersuchungsergebnis nicht negativ beeinflussen. Negative Einflüsse auf das Untersuchungsergebnis können bei der MR dann eintreten, wenn die Patientenliege z. B. metallische oder elektrisch leitfähige Materialien enthält und bei der CT, wenn die Liege aus einem Material gefertigt ist, welches Röntgenstrahlen sehr stark absorbiert.

Aus Gründen der mechanischen Festigkeit und Steifigkeit werden deshalb Patientenliegen für diagnostische Geräte häufig aus Faserverbundwerkstoffen hergestellt.

Patientenliegen aus Faserverbundwerkstoffen sind an sich bekannt. So ist in der DE 38 33 594 A1 eine Liege aus Glasfaserverstärkten Kunststoff (GFK) beschrieben. Die Liege besteht aus einem bidirektional aufgebauten Material mit einem E-Modul von ca. 15 GPa oder aus einem unidirektional aufgebauten Material mit einem E-Modul von ca. 30 GPa. Eine aus diesen Materialien aufgebaute Liege erfüllt die Forderung nach einer hohen Festigkeit und Steifigkeit. Sie ist aber aufgrund der Dichte des Materials von ca. 2 000 kg/m³ sehr schwer. Sie wird in MR-Geräten verwendet, weil GFK ein elektrisch nicht leitfähiger Werkstoff ist. Eine Verwendung in CT-Geräten ist nicht vorgesehen. Der Grund dafür besteht darin, dass Glasfasern eine hohe Absorption für Röntgenstrahlen aufweisen.

Andere Patientenliegen werden aus Carbonfaserverstärkten Kunststoffen (CFK) hergestellt. Eine solche Liege und ihre Verwendung ist in der EP 0 017 454 A1 beschrieben. Solche Liegen sind sehr leicht und haben eine hohe Festigkeit und Steifigkeit. Gleichzeitig ist die Absorption von Röntgenstrahlen sehr gering. Durch die verwendeten Carbonfasern ist die Liege aber elektrisch leitfähig. Die elektrische Leitfähigkeit führt aber bei der Verwendung dieser Liege in MR-Geräten zu Feldverzerrungen des Hauptmagnetfeldes und dadurch zu einer Verschlechterung des Untersuchungsergebnisses des Patienten.

In der DE 197 31 234 A1 ist eine Patientenlagerungsvorrichtung bestehend aus einem Faserverbundwerkstoff beschrieben, zu deren Herstellung Fasern aus aromatischen Polyamide benutzt werden. Die aromatischen Polyamide sind jedoch ein sehr teures Fasermaterial und haben gegenüber Carbon- und Glasfasern eine geringere Steifigkeit und Druckfestigkeit. Das führt dazu, dass bei den für eine Anwendung in diagnostischen Geräten oft notwendigen, sehr geringen Wandstärken daraus hergestellte Patientenliegen nicht die notwendige Steifigkeit besitzen und zudem sehr teuer sind. Darüber hinaus nehmen Polyamide aus der Umgebungsluft Feuchtigkeit auf, die wiederum einen negativen Einfluss auf die elektrischen Eigenschaften des Werkstoffes der Patientenlagerungsvorrichtung hat.

Eine große Anzahl von Patientenliegen sind aus Faserverbundwerkstoffen hergestellt. Ein Faserverbundwerkstoff ist ein aus im Allgemeinen zwei Hauptkomponenten (eine bettende Matrix und verstärkende Fasern) bestehender Mehrphasen- oder Mischwerkstoff. Dabei übernehmen die verstärkenden Fasern und die bettende Matrix sehr spezifische Aufgaben. Durch gegenseitige Wechselwirkungen der beiden Komponenten erhält der Faserverbundwerkstoff höherwertige Eigenschaften als jeder der beiden einzeln beteiligten Komponenten. Das bedingt aber auch, dass die beiden beteiligten Materialien sehr unterschiedliche Eigenschaften haben. So können die Fasern elektrisch leitfähig oder isolierend sein. Zum anderen haben die Fasern eine andere Dichte als die Matrixmaterialien. Diese sehr unterschiedlichen Eigenschaften führen dazu, dass Pätientenliegen entweder nur für ein spezielles diagnostisches Untersuchungsverfahren geeignet sind oder dass das Diagnoseergebnis durch den heterogenen Aufbau der Faserverbundwerkstoffe negativ beeinflusst wird.

Alle derartigen Patientenliegen weisen so den Nachteil auf, dass sie nicht für alle gängigen medizinischen Untersuchungsmethoden, insbesondere MR und CT gleichermaßen geeignet sind.

### Darstellung der Erfindung

Der Erfindung liegt die Aufgabe zugrunde, eine Möglichkeit anzugeben, Patienten für alle gängigen medizinischen Diagnoseverfahren gleichermaßen während der Untersuchungszeit stabil in einer Position zu lagern.

Erfindungsgemäß wird die Aufgabe mit einer Patientenliege dadurch gelöst, dass als Material zur Herstellung der Patientenliege eigenverstärkte Kunststoffe, insbesondere eigenverstärkte Polyolefine und ganz speziell eigenverstärktes Polyethylen verwendet wird. Speziell bändchenförmige, hochverstreckte Monofilamente besitzen eine Reihe von Eigenschaften, die sich sehr verteilhaft für die Herstellung von Patientenliegen für diagnostische Geräte auswirken.

Sie zeichnen sich dadurch aus, dass sie bei einer Dichte < 1 000 kg/m³ je nach Verstreckungsgrad und verwendetem Polyethylen einen E-Modul von mehr als 30 GPa, teilweise bis zu 70 GPa und höher aufweisen. Der Verstreckungsgrad kann dabei das 15- bis 60-fache betragen. Gleichzeitig sind sie elektrisch nicht leitend und zeigen eine sehr geringe Absorption von Röntgenstrahlen sowie eine extrem geringe Feuchtigkeitsaufnahme.

Zur Herstellung von Bauteilen aus diesen Materialien können die bändchenförmigen, hochverstreckten Monofilamente mit arteigenen Kunststoffen gefügt werden. Dieser Fügevorgang kann Prinzip des Schmelzklebens oder des Sinterns entsprechen. Der Anteil der bändchenförmigen, hochverstreckten Monofilamente in solchen Verbundkörpern ist deutlich größer als 95 %. Die so hergestellten Verbundkörper können durch gezielte Orientierung der bändchenförmigen Monofilamente im Bauteil an spezielle Anforderungen des jeweiligen Gerätes angepasst werden.

So können in einer speziellen Ausgestaltüngsform die Monofilamente zu gleichen Anteilen in 0°-(Längs-), 90°-(Querrichtung) und ±45°-Richtung der Patientenliege angeordnet sein. Die Liege hat dann in allen Richtungen gleiche Eigenschaften und kann deshalb Längs-, Quer- und Torsionsbelastungen gleich gut Stand halten.

Es ist aber auch möglich die Monofilamente ausschließlich in 0°-Richtung anzuordnen. Eine derartig hergestellte Patientenliege zeichnet sich durch eine besonders hohe Festigkeit und Steifigkeit in Längsrichtung aus.

Weiterhin ist es möglich, die Monofilamente als Deckschicht für einen Sandwichaufbau zu verwenden. Als Kern für einen solchen Sandwich kann jeder handelsübliche Hartschaumstoff verwendet werden. Eine derartig hergestellte Patientenliege zeichnet sich durch ihr extrem geringes Gewicht aus.

### Weg(e) zur Ausführung der Erfindung

Die Erfindung soll nachfolgend anhand eines Ausführungsbeispiels näher erläutert werden. Zur Herstellung einer erfindungsgemäßen Patientenliege wurde Kaypla® 525H Unidrectional HDPE (Teijin Monofilament Germany GmbH, Deutschland) mit einem Flächengewicht von 105 g/m² verwendet. Das Material hat einen E-Modul von 50 GPa und eine Dichte von < 950 kg/m³. Zum Aufbau der Patentenliege wurden einzelne Lagen dieses Materials auf eine Negativform schichtweise drapiert. Der Schichtaufbau besteht in dieser Ausführung aus einem symmetrischen Aufbau von außen beginnend aus 1 Lage in Längsrichtung (0°-Richtung), 12 Lagen abwechselnd +45°- und -45°-Richtung, 4 Lagen in 90°-Richtung und weiteren 40 Lagen wieder in 0°-Richtung bis zur Bauteilmitte. Der Lagenaufbau wiederholt sich nun von innen nach außen in umgekehrter Reihenfolge, d. h. 40 Lagen in 0°-Richtung, 4 Lagen in 90°-Richtung, 12 Lagen abwechselnd +45°- und -45°-Richtung und abschließend 1 Lage in 0°-Richtung. Das gesamte Bauteil besteht somit aus 114 Einzellagen. Anschließend wird das so aufgebaute Lagenpaket in einen Foliesack eingepackt, wie er typisch ist für den Vakuumaufbau eines Glas- oder Carbonfaseiprepregs für eine Autoklavenaushärtung. Das gesamte Paket wird bei einem Druck von bis zu 2 mbar evakuiert, in einen Autoklaven verbracht und bei einem Autoklavendruck von 9,5 bar und bei einer Temperatur von 130 °C thermogeformt. Nach 2 Stunden erfolgt eine Abkühlung des Lagenpaketes unter Druck auf unter 65 °C. Anschließend kann das Bauteil entformt werden. Das fertige Bauteil hat eine Dicke von 12 mm und ein Gewicht von ca. 11,5 kg/m². Die so hergestellte Patientenliege hat eine ausgezeichnete Steifigkeit und Festigkeit und zeigt in der Verwendung sowohl in der Magnetresonanzspektroskopie als auch in der Computertomographie keine störenden Einflüsse auf die Abbildungsqualität der bildgebenden Analyse.

## Patentansprüche

1. Patientenliege für diagnostische Geräte, **dadurch gekennzeichnet, dass** die Patientenliege aus bändchenförmigen hochverstreckten Monofilamenten besteht, wobei alle diese Monofilamente mindestens eine Orientierung aufweisen und über einen arteigenen Kunststoff gefügt sind.

2. Patientenliege nach Anspruch 1, **dadurch gekennzeichnet, dass** der arteigene Kunststoff aus der Gruppe der Polyolefine ausgewählt ist.

3. Patientenliege nach Anspruch 2, **dadurch gekennzeichnet, dass** das Polyolefin Polyethylen hoher Dichte (PE-HD) ist.

4. Patientenliege nach Anspruch 3, **dadurch gekennzeichnet, dass** das Polyethylen hoher Dichte aus hochorientierten Polyethylenbändchen besteht.

5. Verfahren zur Herstellung einer Patientenliege nach Anspruch 5, **dadurch gekennzeichnet, dass**
- die Monofilamente auf eine Form schichtweise übereinandergestapelt werden,
- das aufgebaute Lagenpaket mit Form in einen Foliensack eingepackt wird,
- danach der Foliensack mit einem Druck bis zu 2 mbar evakuiert wird,
- anschließend der Foliensack in einen Autoklaven verbracht und über 2 Stunden mit einem Autoklavendruck von 9,5 bar und einer Temperatur von 130°C thermogeformt wird und
- abschließend abgekühlt und entformt wird.

6. Verfahren zur Herstellung einer Patientenliege nach Anspruch 5, **dadurch gekennzeichnet, dass** die Thermoformung in einem geschlossenen, auf 130°C aufgeheizten Werkzeug in einer Presse bei einem Pressdruck von ca. 10 bar und einer Presszeit von 2 Stunden erfolgt.

7. Verfahren zur Herstellung einer Patientenliege nach Anspruch 5, **dadurch gekennzeichnet, dass** als Form eine Negativform verwendet wird.

8. Verfahren zur Herstellung einer Patientenliege nach Anspruch 5 oder 6, **dadurch gekennzeichnet, dass** die Monofilamente in 0°-Richtung, 90°-Richtung und ±45°-Richtung wechselnd auf die Form schichtweise übereinander gestapelt werden.

## Claims

1. Patient table for diagnostic equipment, **characterized in that** the patient table comprises ribbon-shaped, highly stretched monofilaments, wherein all these monofilaments have at least one orientation and are joined with a type-specific plastic.

2. Patient table according to claim 1, **characterized in that** the type-specific plastic is selected from the group of polyolefins.

3. Patient table according to claim 2, **characterized in that** the polyolefin is high-density polyethylene (HDPE).

4. Patient table according to claim 3, **characterized in that** the high-density polyethylene comprises highly oriented polyethylene ribbons.

5. Method for the production of a patient table according to claim 5, **characterized in that**
- the monofilaments are stacked one on top of the other in layers on a mold,
- the formed layer assembly with mold is packed into a foil bag,
- the foil bag is then evacuated at a pressure of up to 2 mbar,
- the foil bag is then inserted into an autoclave and thermoformed for 2 hours at an autoclave pressure of 9.5 bar and a temperature of 130 °C and
- is then cooled and removed from the mold.

6. Method for the production of a patient table according to claim 5, **characterized in that** the thermoforming is carried out in a closed die heated to 130 °C in a press at a pressing pressure of approximately 10 bar and a pressing time of 2 hours.

7. Method for the production of a patient table according to claim 5, **characterized in that** a female mold is used as mold.

8. Method for the production of a patient table according to claim 5 or 6, **characterized in that** the monofilaments are stacked one on top of the other in layers on the mold alternately in 0° direction, 90° direction and ±45° direction.

## Revendications

1. Table médicale pour des appareils diagnostiques, **caractérisée en ce que** la table médicale comprend des monofilaments fortement étirés en forme de bandelettes, tous ces monofilaments ayant au moins une orientation et étant assemblés par un plastique propre à l'espèce.

2. Table médicale selon la revendication 1, **caractérisée en ce que** le plastique propre à l'espèce est sélectionné du groupe des polyoléfines.

3. Table médicale selon la revendication 2, **caractérisée en ce que** la polyoléfine est du polyéthylène haute densité (PEHD).

4. Table médicale selon la revendication 3, **caractérisée en ce que** le polyéthylène haute densité comprend des bandelettes de polyéthylène fortement orientées.

5. Procédé pour la fabrication d'une table médicale selon la revendication 5, **caractérisé en ce que**
- les monofilaments sont empilés les uns sur les autres par couches sur un moule,
- l'assemblage de couches formé avec moule est mis dans un sac en feuille,
- puis, le sac en feuille est évacué à une pression de jusqu'à 2 mbar,
- puis, le sac en feuille est mis dans un autoclave et thermoformé sur deux heures à une pression d'autoclave de 9,5 bar et une température de 130 °C et
- finalement est refroidi et démoulé.

6. Procédé pour la fabrication d'une table médicale selon la revendication 5, **caractérisé en ce que** le thermoformage est effectué dans un moule fermé chauffé à 130 °C dans une presse à une pression de pressage d'environ 10 bar et une durée de pressage de 2 heures.

7. Procédé pour la fabrication d'une table médicale selon la revendication 5, **caractérisé en ce qu'**un moule femelle est utilisé comme moule.

8. Procédé pour la fabrication d'une table médicale selon la revendication 5 ou 6, **caractérisé en ce que** les monofilaments sont empilés les uns sur les autres par couches sur le moule de manière alternante en direction de 0°, en direction de 90° et en direction de ±45°.
